# EUROPEAN PATENT APPLICATION

(11) **EP 2 323 055 A1**
(43) Date of publication of application: **18.05.2011**
(21) Application number: 08791084.0
(22) Date of filing: 11.07.2008
(51) Int. Cl.: G06F 19/00

(54) **CHARACTER EXTRACTION PROCESS, CHARACTER EXTRACTION APPARATUS AND CHARACTER EXTRACTION PROGRAM**

(71) Applicant: NEC Soft, Ltd., Tokyo 136-0082 (JP)
(72) Inventor: AKITOMI, Jou, Tokyo 136-0082 (JP)
(74) Representative: Vossius & Partner
(86) International application number: PCT/JP2008/062603
(87) International publication number: WO 2010/004650

(57) **Abstract**

Provided are a feature extraction method of creating a feature vector for objectively evaluating the sequence of aptamer on the basis of the biological features and a feature extraction apparatus and a feature extraction program for performing the method. The feature extraction method according to the present invention includes a step of predicting a secondary structure of a base sequence applied and a step of creating a feature vector based on a predicted secondary structure of the sequence.

SEQUENCE LISTING

<110>  NEC Soft, Ltd.

<120>  Feature extraction method, feature extraction apparatus, and feature
extraction program

<130>  T1241 EP

<140>  EP 08 79 1084.0
<141>  2008-07-11

<160>  4

<170>  PatentIn version 3.4

<210>  1
<211>  16
<212>  RNA
<213>  Artificial sequence

<220>
<223>  /note="Description of artificial sequence: for Embodiment 1"

<400>  1
ggcgcauuau gcagcc                                                    16


<210>  2
<211>  20
<212>  RNA
<213>  Artificial sequence

<220>
<223>  /note="Description of artificial sequence: for Embodiment 2"

<400>  2
aaaacgcgau aacgcgaauu                                                20


<210>  3
<211>  26
<212>  RNA
<213>  Artificial sequence

<220>
<223>  /note="Description of artificial sequence: for Embodiment 3"

<400>  3
aaacccgaaa ggguuuuuga gaaaaa                                         26


<210>  4
<211>  26
<212>  RNA
<213>  Artificial sequence

<220>
<223>  /note="Description of artificial sequence: for Embodiment 3"

<400>  4
cccccgagag ggguuuugcc cuugcc                                         26

## Description

### Technical Field

The present invention relates to a feature extraction method, and also relates to a feature extraction apparatus and a feature extraction program for performing the method. In particular, the present invention relates to a feature extraction method considering the biological features of nucleotides such as aptamer, and relates to a feature extraction apparatus and a feature extraction program for performing the method.

### Background Art

It has been considered that nucleotides such as DNA and RNA mainly have functions as molecular species involved in the synthesis of proteins. However, in recent years, the phenomenon in which, by direct interactions between nucleotides such as ribozyme, RNAi, and aptamer and molecular species such as proteins and polymers, the functions of the molecular species can be controlled has been found and receiving attention. Among the foregoing nucleotides, aptamer is obtained, for example, by the Systematic Evolution of Ligands by EXponential enrichment (SELEX) method.

For example, in the case of trying to obtain aptamer by the SELEX method, generally, plural sequences are simultaneously obtained. When the plural sequences of aptamer are obtained in this manner, in many cases, the first thing to be performed by researchers is the classification of the sequences. Except for the case in which the sequences enriched to the extent where there is no margin for classification are obtained, researchers should classify the sequences obtained with some criteria. In the case of correctly classifying plural base sequences, there are many considerations, i.e., not only simply similarities of sequences but also similarities of secondary structures should be considered. As the method of classifying the sequences obtained, there is a method of classifying on the basis of the presence or absence of a short base sequence that is called a motif locally stored between sequences. However, there are often the cases where the detection of motif in a pool obtained is difficult or plural motifs are present. In such cases, there is a problem that it is difficult to perform the foregoing classification method and the classification is often performed subjectively on the basis of the experiences of researchers.

Further, researchers generally classify the plural sequences obtained, summarize the data into classes, and evaluate each of them as the aptamer having the same function.
Generally, it is expected that the binding capacity or the binding mode relative to a target molecule differs among the classes. However, since the foregoing classification is performed based on the own judgment of researchers by observing the sequences, it is highly likely that classes classified in this manner differ from the actual functional classes.

In this manner, in the classification of the sequences, there has been a demand for the method of objectively evaluating the properties.

The applicant could not find any published document relating to the present invention at the time of the filing of the present application. Therefore, no related art document is disclosed.

### Disclosure of the Invention

### Problem to be Solved by the Invention

The present invention is made in view of the foregoing conventional problems. The present invention is intended to provide a feature extraction method of creating a feature vector for objectively evaluating the sequence of aptamer on the basis of the biological features, and to provide a feature extraction apparatus and a feature extraction program for performing the method.

### Means for Solving Problem

The feature extraction method according to the present invention includes the steps of: predicting a secondary structure of a base sequence applied; and creating a feature vector based on a predicted secondary structure of the sequence.

The feature extraction apparatus according to the present invention includes: a secondary structure prediction unit for predicting a secondary structure of a base sequence applied; and a feature vector creation unit for creating a feature vector based on a predicted secondary structure of the sequence.

The feature extraction program according to the present invention performs the steps of: predicting a secondary structure of a base sequence applied; and creating a feature vector based on a predicted secondary structure of the sequence.

### Effects of the Invention

According to the present invention, the feature vector based on the biological features of nucleotides such as aptamer can be obtained. As a result, by applying general pattern recognition algorithms such as a support vector machine and a K-means relative to the obtained feature vector, the problem of the classification of the base sequences of aptamer can be solved and also the possibilities of applying the sequences to various applications of the statistical analysis may occur.

The foregoing can be achieved because the feature vector that is the base of the statistical analysis method can be provided on the basis of the biological feature that aptamer binds to a target molecule at a loop site of the secondary structure.

### Brief Description of Drawings

FIG. 1 is a schematic view showing the configuration of the feature extraction apparatus according to the present invention.
FIG. 2 is a flowchart showing the operation of each step of the feature extraction method according to the present invention.
FIG. 3-1 is an example of a flowchart showing the operation of the step of creating a feature vector on the basis of the secondary structure in the feature extraction method according to the present invention.
FIG. 3-2 is another example of a flowchart showing the operation of the step of creating a feature vector on the basis of the secondary structure in the feature extraction method according to the present invention.
FIG. 3-3 is yet another example of a flowchart showing the operation of the step of creating a feature vector on the basis of the secondary structure in the feature extraction method according to the present invention.
FIG. 4 is a flowchart showing the operation of calculating/storing a weighted feature vector.
FIG. 5 is a view showing the sequence and the secondary structure thereof used in Example 1.
FIG. 6 is a view showing the process of deriving a feature vector in Example 1.
FIG. 7 is a view showing the sequence and the structures thereof used in Example 2.
FIG. 8 is a view showing the process of deriving a feature vector in Example 2.
FIG. 9 is a view showing the sequences and the structures thereof used in Example 3.
FIG. 10 is a view showing the process of deriving feature vectors in Example 3.

### Explanation of Reference Numerals

- 1: input device
- 2: data processing device
- 3: storage device
- 4: output device
- 21: secondary structure prediction unit
- 22: feature vector creation unit
- 31: sequence storage unit
- 32: secondary structure storage unit

### Best Mode for Carrying Out the Invention

Hereinafter, the preferred embodiment according to the present invention will be explained.

Hereinafter, the feature extraction method, the feature extraction apparatus, and the feature extraction program according to the present invention will be explained in detail with reference to the schematic view of the feature extraction apparatus according to the present invention.

The feature extraction apparatus according to the present invention is the apparatus that performs the feature extraction method according to the present invention. The feature extraction program according to the present invention is the program that performs the feature extraction method according to the present invention. Further, in the present invention, the term, "base sequence", means sequences of various genes such as DNA and RNA.

### (Configuration of Feature Extraction Apparatus according to Present Invention)

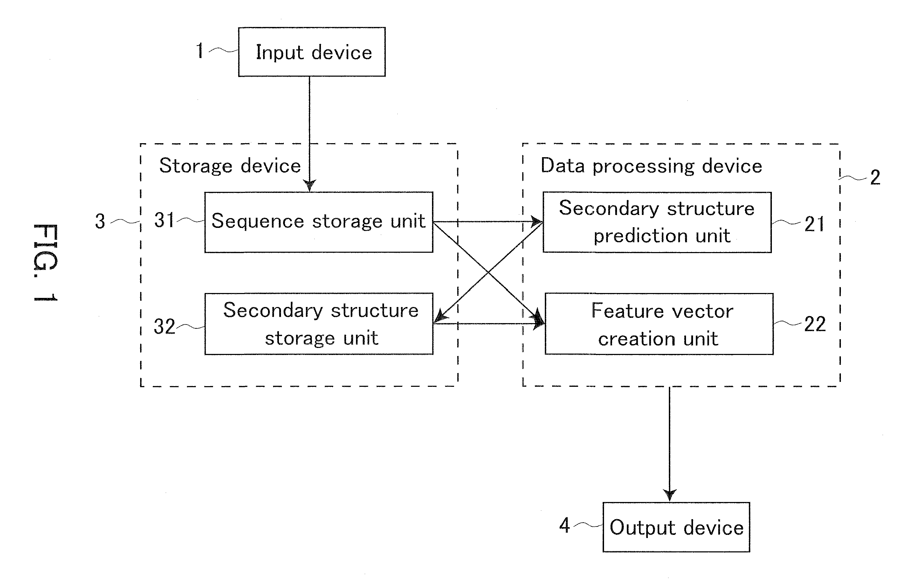

FIG. 1 is a schematic view showing the configuration of the feature extraction apparatus according to the present invention. With reference to FIG. 1, the feature extraction apparatus according to the present invention includes an input device 1 such as a keyboard, a data processing device 2 operated by program control, a storage device 3 storing information, and an output device 4 such as a display device and a printing device.

The data processing device 2 includes a secondary structure prediction unit 21 and a feature vector creation unit 22.

The secondary structure prediction unit 21 retrieves the information of a base sequence of aptamer or the like from a sequence storage unit 31 and predicts the secondary structure of the sequence. The method of predicting the secondary structure of the sequence is not particularly limited and is applicable as long as the secondary structure of the base sequence can be predicted, and an example thereof includes mfold that is a method utilizing experimental thermodynamic parameters. The information of the secondary structure of the sequence predicted by the foregoing method is stored in a secondary structure storage unit 32.

The feature vector creation unit 22 retrieves the information of the base sequence from the sequence storage unit 31 and retrieves the information of the predicted secondary structure from the secondary structure storage unit 32, and extracts only the bases of loop sites not forming base pairs from the base sequence. Then, with respect to the bases of the loop site extracted, it is searched whether or not a specific base pattern is present. For example, "1" is applied and stored as the one-dimensional value of the feature vector when the specific base pattern is present and "0" is applied and stored as the one-dimensional value of the feature vector when the specific base pattern is not present. This operation is repeated as long as there is the base pattern that should be searched, and in accordance with this, the number of dimensions of the feature vector increases. After the completion of the repetition, the feature vector is sent to the output device 4.

The storage device 3 includes the sequence storage unit 31 and the secondary structure storage unit 32.

The sequence storage unit 31 stores the base sequence of aptamer or the like applied by the input device 1.

The secondary structure storage unit 32 stores the secondary structure of the base sequence predicted by the secondary structure prediction unit 21.

### (Steps of Feature Extraction Method according to Present Invention and Operations of Feature Extraction Apparatus and Feature Extraction Program according to Present Invention)

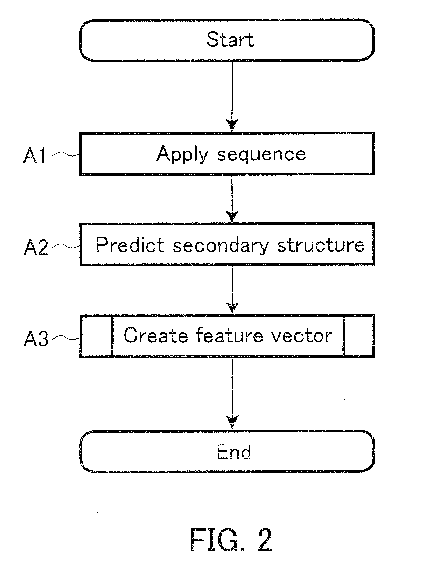

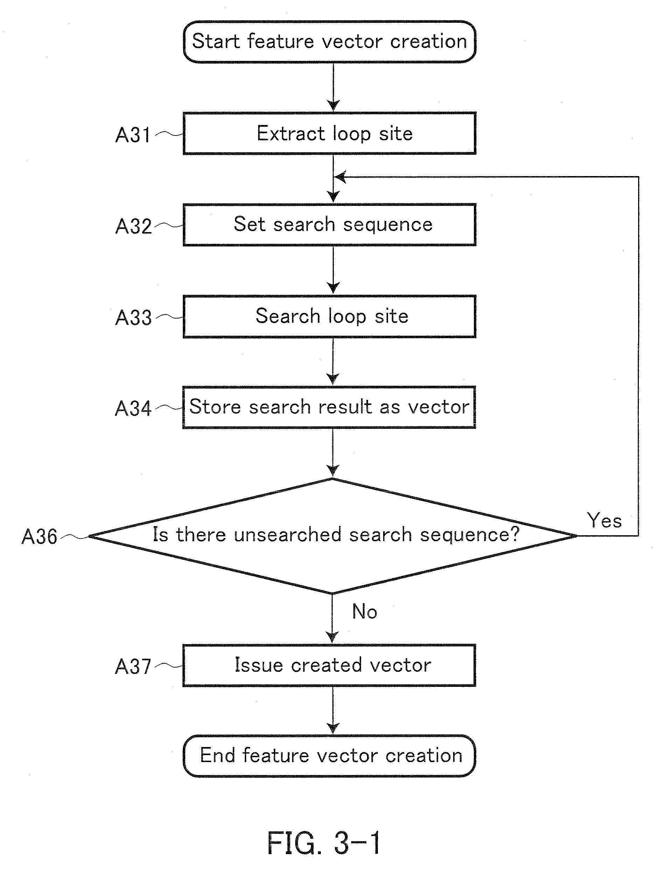

Next, the steps of the feature extraction method according to the present invention and the operations of the feature extraction apparatus and the feature extraction program according to the present invention will be explained in detail with reference to the schematic view of FIG. 1 and the flowcharts of FIGs. 2 and 3-1.

The base sequence applied by the input device 1 is stored in the sequence storage unit 31. Further, the secondary structure of this base sequence is predicted by the secondary structure prediction unit 21, and the predicted secondary structure is stored in the secondary structure storage unit 32 and sent to the feature vector creation unit 22 (A1, A2, and A3).

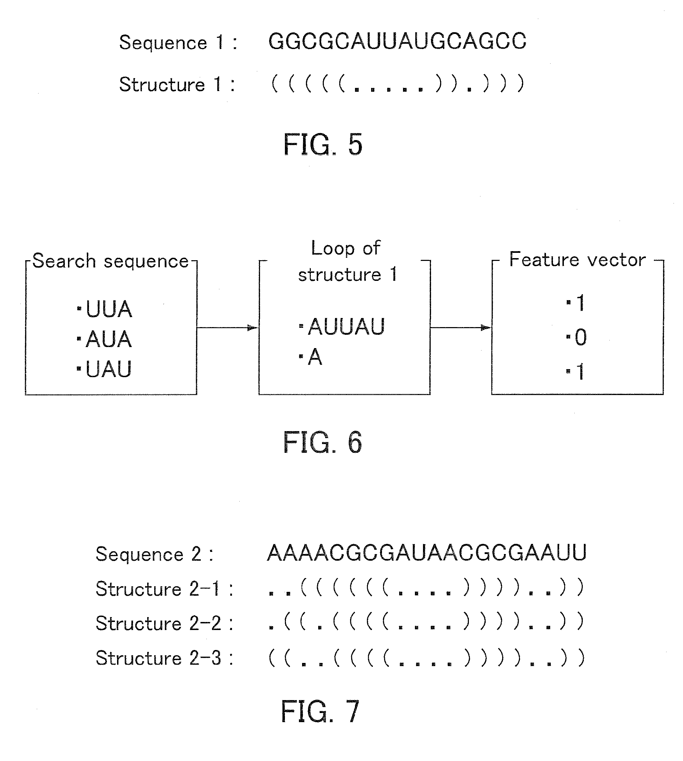

The feature vector creation unit 22 retrieves the information of the base sequence stored in the sequence storage unit 31 and retrieves the information of the secondary structure of the sequence stored in the secondary structure storage unit 32, and extracts loop sites by removing the bases forming base pairs from the base sequence (A31). Here, the method of extracting a loop site is not particularly limited. For example, as shown in FIG. 5, the following method may be employed: bases forming base pairs are expressed as "(" and ")" and the other bases are expressed as ".", and the bases corresponding to "(" and ")" are removed. The thus extracted loop sites serve as the targets to be searched by a search sequence in the later step. In the feature vector creation unit 22, the bases forming base pairs in the base sequence applied may serve as the targets to be searched by a search sequence in the later step.

Next, the feature vector creation unit 22 sets a search sequence for searching a loop site (A32). As described below, this search sequence may be formed by the feature vector creation unit 22 as required. Then, the feature vector creation unit 22 searches the loop site using this search sequence (A33). As a result of the search, for example, "1" is stored as the one-dimensional value of the vector when the search sequence is present in the loop site and "0" is stored as the one-dimensional value of the vector when the search sequence is not present in the loop site.

The search sequence may be an arbitrary number of base patterns arbitrarily selected by the user or all base patterns that could be present in an appropriate range may be searched in the round-robin fashion. In the case where all the base patterns are searched in the round-robin fashion, the search sequence formed and set (A32) may be expressed by four kinds bases (A, C, G, and U or T in the case of RNA) or may be expressed by the combination of five letters in which "." for a mismatch is added to the foregoing four bases. The mismatch may be understood as the one that can take any base of A, C, G, and U or T or the one that has been truncated. Further, the mismatch may be understood as the arbitrary combination of the bases, A, C, G, and U or T and the base that has been truncated, namely, "G and C" and "A and U". Accordingly, the possible combination in the case where the search sequence is composed of five bases is the 5^{th} power of 4 from AAAAA to UUUUU and the number of the combinations further increases as accepting mismatches of bases.

After the storage of the feature vector of the search result, when the unsearched search sequence is present, the steps from the setting of the search sequence (A32) to the storage of the search result (A34) are repeated and the result is sequentially stored as the next dimensional value of the feature vector. Accordingly, the number of search sequences is the dimensional number of feature vector.

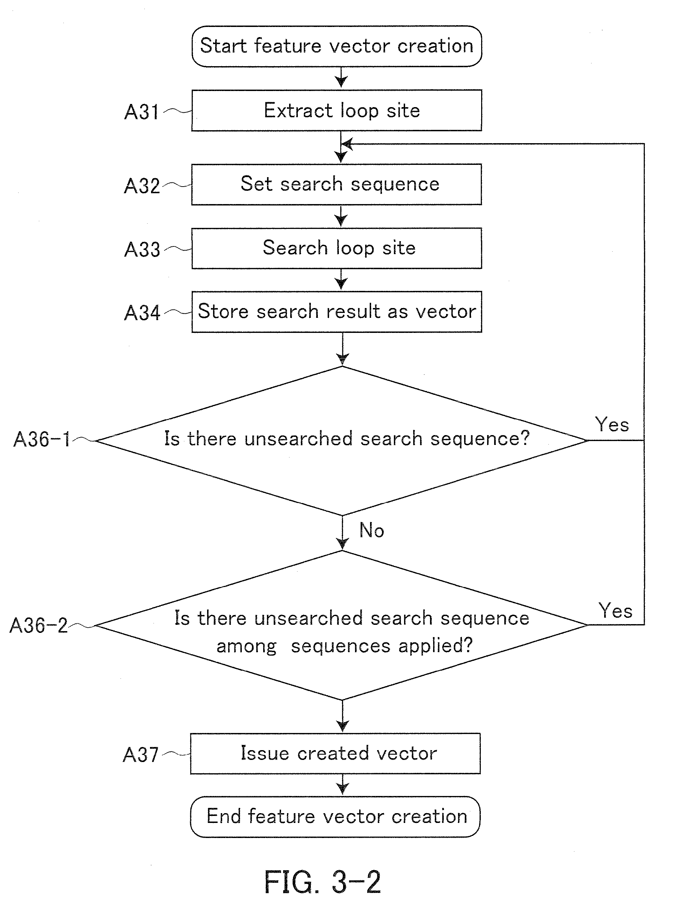

In the case where plural sequences are applied in the step A1, as shown in FIG. 3-2, first, the steps from the setting of the search sequence to the storage of the search result (A32-A34) are repeated with respect to a sequence until there is no more unsearched search sequences. When there is no more unsearched search sequences (corresponding to "No" in A36-1), the same steps as described above are repeated with respect to the next sequence (A36-2 to A 36-1).

When plural sequences are applied and the search results are stored as the feature vector as described above, weights may be assigned to the search results in order of importance of these plural sequences. For example, with respect to the plural sequences applied, in accordance with the number of clones of the sequence obtained, the values obtained by multiplying the search results by the number of clones may be stored in the feature vector as the weighted feature vector.

As the method of assigning weights, the method of simply summing respective dimensional components may be employed. Further, the following method may be employed: a weight vector is calculated on the basis of the feature vector obtained by searching a loop site, weights are assigned to the previously obtained feature vector on the basis of the feature vector and the weight feature vector, and the resultant is stored as the finally obtained feature vector.

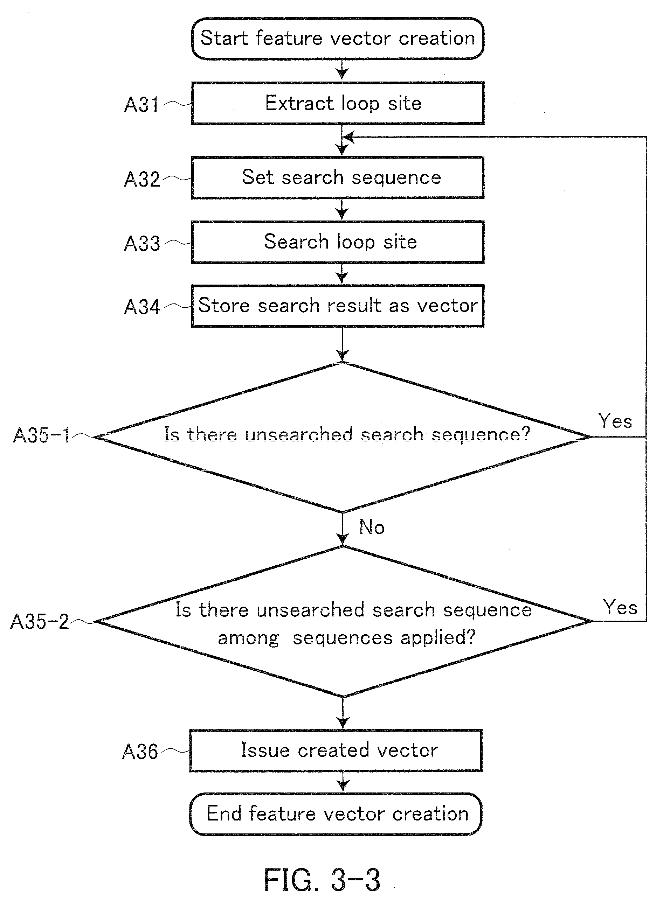

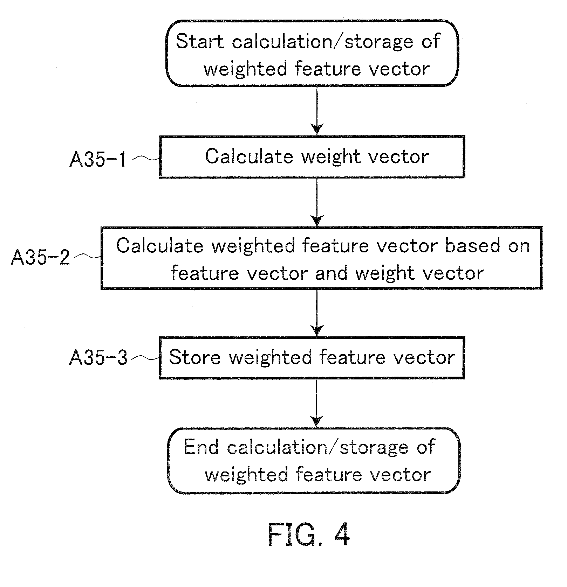

FIG. 3-3 is an example of a flowchart showing the operation of the step of creating a feature vector on the basis of the secondary structure in the feature extraction method according to the present invention. FIG. 4 is a flowchart showing the operation of calculating/storing a weighted feature vector. In FIG. 3-3, the steps are the same as those described above except for the step of calculating/storing a weighted feature vector (A35).

With reference to FIG. 4, in the step of calculating/storing the weighted feature vector, first, the feature vector creation unit 22 calculates the weight vector on the basis of the feature vector obtained before A34 by searching the loop site in which the search sequence is present (A35-1). Here, as the method of calculating the weight vector from the feature vector, the following method may be employed: with respect to all the feature vectors obtained, summing is performed for every dimensional components.

Next, the feature vector creation unit 22 calculates the weighted feature vector on the basis of the previous feature vector and the weight vector obtained as described above (A35-2). Here, as the method of assigning weights to the feature vector previously obtained, the method of multiplying each dimensional component of the feature vector by the corresponding dimensional component of the weight vector may be employed. Thereafter, the feature vector creation unit 22 stores the weighted feature vector (A35-3). Here, as the method of storing, the weighted feature vector may be stored as a new feature vector in place of the previous feature vector or the weighted feature vector may be stored with the previous feature vector. Accordingly, in the case where the weighted feature vector is stored with the previous feature vector, two feature vectors, namely the feature vector to which weights have been assigned and the feature vector to which weights have not been assigned, are stored.

On the other hand, in the case where plural secondary structures are predicted with respect to a sequence, it may comprehensively be searched whether or not the search sequence set is included in the loop sites of the secondary structures with respect to all the plural secondary structures. For example, in the case where plural secondary structures are predicted with respect to a sequence, it may be searched whether or not the search sequence is present with respect to all the loop sites extracted with respect to the plural secondary structures, and the result thus obtained may be treated as a feature vector of the sequence. Alternatively, a feature vector may be obtained for every secondary structure with respect to a sequence. In this case, in order of importance of the secondary structure, weights may be assigned to the feature vector. For example, the result obtained by multiplying the feature vector by the thermodynamic parameter of the secondary structure may be treated as the feature vector.

Further, in the case where one or plural sequences are applied or in the case where one or plural secondary structures are predicted with respect to one or plural sequences, the assignment of weights may be performed as described above. The assignment of weights may be performed by calculating the number of loop sites having a search sequence.

When the search of all the search sequences is completed with respect to all the sequences applied, the feature vector created is issued by the output device 4 (A36).

### Examples

The present invention will be explained with specific examples.

### (Example 1)

First, when the base sequence (sequence 1) as shown in FIG. 5 is applied from the input device 1, the sequence is stored in the sequence storage unit 31 (A1). The secondary structure prediction unit 21 predicts the secondary structure of the sequence. As the result of the prediction, when the prediction result of the secondary structure (structure 1) as shown in FIG. 5 is obtained, the result is stored in the secondary structure storage unit 32 (A2). The sequence applied and the secondary structure thereof are sent to the feature vector creation unit 22 (A3).

The feature vector creation unit 22 first extracts a loop site not forming a base pair in the sequence from the sequence and the secondary structure (A31), and in the present case, the loop sites as shown in FIG. 6 are obtained. Next, the search sequence for searching the loop sites is set (A32). In the case where three search sequences are prepared as shown in FIG. 6, first of all, the search is performed with respect to the first sequence "UUA" (A33). Since "UUA" is included in "AAUAU" out of the "AUUAU" and "A", which are the loop sites to be searched, the result that the search sequence is present in the loop site is stored as one-dimensional value of the feature vector. In the case where "1" is stored when the search sequence is present in the loop site and "0" is stored when the search sequence is not present in the loop site, since the present case corresponds to the case in which the search sequence is present in the loop site, "1" is stored as the one-dimensional value of the feature vector (A34). This is the last step of searching the first sequence. Next, since there are still two unsearched search sequences, the steps from A32 to A34 are repeated in the same manner as described above (A36). The second search sequence "AUA" is not present in the loop site and the third search sequence "UAU" is present in the loop site. Therefore, as the search result, the feature vector {1, 0, 1} as shown in FIG. 6 is obtained. This feature vector is issued by the output device 4 (A 37). This is the feature vector obtained according to the present invention showing the features of the base sequence shown in FIG. 5.

### (Example 2)

First, when the base sequence (sequence 2) as shown in FIG. 7 is applied from the input device 1, the sequence is stored in the sequence storage unit 31 (A1). The secondary structure prediction unit 21 predicts the secondary structure of the sequence. As the result of the prediction, when the prediction results of the secondary structures (structures 2-1 to 2-3) as shown in FIG. 7 are obtained, these are stored in the secondary structure storage unit 32 (A2). The sequence applied and the secondary structures thereof are sent to the feature vector creation unit 22 (A3).

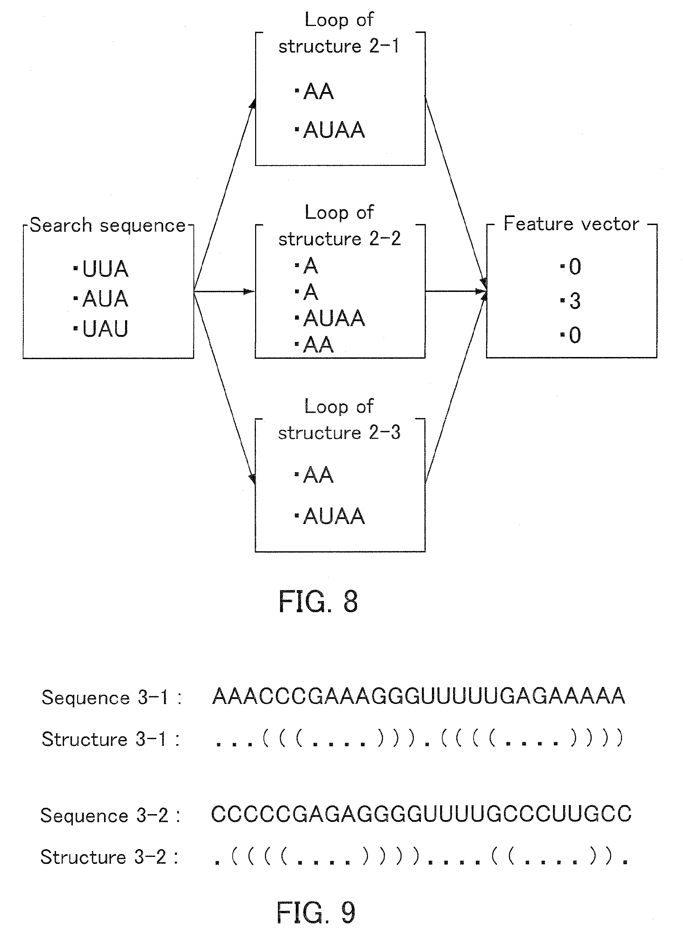

The feature vector creation unit 22 first extracts a loop site not forming a base pair in the sequence from the sequence and a secondary structure out of the predicted results (A31), and the extraction is performed in the same manner as described above with respect to all the prediction results of the secondary structures. In the present case, the loop sites as shown in FIG. 8 are obtained. Next, the search sequence for searching the loop sites is set (A32). In the case where three search sequences are prepared as shown in FIG. 8, first of all, the search is performed with respect to the first sequence "UUA" (A33). "UUA" is not included in "AA" or "AUAA", which is the loop site derived from a predicted secondary structure (structure 2-1) among the loop sites to be searched. Next, "UUA" is not included in "A", "AUAA", or "AA", which is the loop site derived from a predicted secondary structure (structure 2-2) among the loop sites to be searched. Next, "UUA" is not included in "AA" or "AUAA", which is the loop site derived from a predicted secondary structure (structure 2-3) among the loop sites to be searched. These results show that the search sequence "UUA" is not included in any of the loop sites derived from the predicted secondary structures. In the case where "1" is stored when the search sequence is present in the loop site and "0" is stored when the search sequence is not present in the loop site as described above, since the present case corresponds to the case in which the search sequence is not present in the loop site, "0" is stored as the one-dimensional value of the feature vector (A34). In this manner, the search is performed with respect to a search sequence until there is no more loop sites that should be searched, and then the search for the first sequence is completed. Next, since there are still two unsearched search sequences, the steps from A32 to A34 are repeated in the same manner as described above (A36). The second search sequence "AUA" is present in the structure 2-1-derived "AUAA", the structure 2-2-derived "AUAA", and the structure 2-3 derived "AUAA" out of the loop sites derived from the predicted secondary structures (structures 2-1 to 2-3). Further, the third search sequence "UAU" is not present in any of the loop sites derived from the predicted secondary structures (structures 2-1 to 2-3). In Example 2, when the assignment of weights to the search sequence "AUA" is performed by summing the number of loop sites in which the search sequence is present, as the result of the assignment of weights, the feature vector {0, 3, 0} as shown in FIG. 8 is obtained. This feature vector is issued by the output device 4 (A 37). This is the feature vector obtained according to the present invention showing the features of the base sequence shown in FIG. 7.

In Example 1, the number of secondary structures predicted by the secondary structure prediction unit 21 is one (A2). Example 2 corresponds to the example in which, in the case where plural suboptimal secondary structures are present, they also are stored in the secondary structure storage unit 32 and loop sites are extracted therefrom as additional loop sites at the time of extracting the loop site by the feature vector creation unit 22 (A31). In this case, since the number of patterns of the loop site virtually increases, the probability of the presence of the search sequence increases. The feature vector obtained in the example of this case has high sensitivity although the specificity thereof is inferior to that of the original feature vector. In Example 2, the value obtained by summing the appearance frequency is stored as the feature vector. However, the assignment of weights may be performed for every dimensions of the vector by other methods. Further, in accordance with weights, by eliminating the low-weighted-dimensions of the feature vector, the redundant dimensions of the feature vector may be compressed.

### (Example 3)

First, when the base sequences (sequences 3-1 to 3-2) as shown in FIG. 9 are applied from the input device 1, the sequences are stored in the sequence storage unit 31 (A1). The secondary structure prediction unit 21 predicts the secondary structures of the sequences. As the result of the prediction, when the prediction results of the secondary structures (structures 3-1 to 3-2) as shown in FIG. 9 are obtained, these are stored in the secondary structure storage unit 32 (A2). The sequences applied and the secondary structures thereof are sent to the feature vector creation unit 22 (A3).

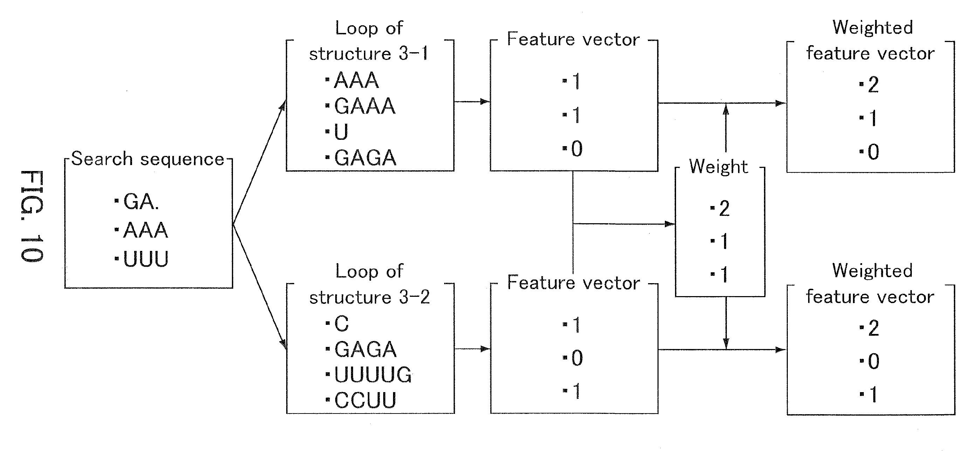

The feature vector creation unit 22 first extracts a loop site not forming a base pair in the sequence from a sequence (sequence 3-1) out of the plural sequences (sequences 3-1 to 3-2) and the prediction result of the secondary structure thereof (structure 3-1), and the extraction is performed in the same manner as described above with respect to all the results of the secondary structures predicted with reference to the plural sequences (A31). In the present case, the loop sites as shown in FIG. 10 are obtained with respect to the respective sequences. Next, the search sequence for searching the loop sites is set (A32). In the case where three search sequences are prepared as shown in FIG. 10, first of all, the search is performed with respect to the first sequence "GA." (A33). "GA." is included in "GAAA" and "GAGA" among the loop sites derived from a targeted sequence (sequence 3-1). In the case where "1" is stored when the search sequence is present in the loop site and "0" is stored when the search sequence is not present in the loop site as described above, in the present case, "1" is stored as the one-dimensional value of the feature vector regardless of the number of loop sites in which the search sequence is present (A34). This is the last step of searching the first sequence with respect to the first search sequence. Next, since there are still two unsearched search sequences (corresponding to "Yes" in A36-1), the steps from A32 to A34 are repeated in the same manner as described above. Since the second search sequence "AAA" is included in "AAA" and "GAAA" among the loop sites derived from a targeted sequence (sequence 3-1), "1" is stored as the two-dimensional value of the feature vector in the same manner as described above (A 34). Further, since the third search sequence "UUU" is not present in any of the loop sites derived from a targeted sequence (sequence 3-1), "0" is stored as the three-dimensional value of the feature vector (A 34). As a result, the feature vector {1, 1, 0} as shown in FIG. 10 is obtained with respect to the sequence (sequence 3-1).

Next, since there is an unsearched sequence (sequence 3-2) among the sequences applied (corresponding to "Yes" in A36-2), the steps from the setting of the search sequence for searching the loop sites derived from this sequence (A32) to the storage of the search result as the feature vector (A34) are repeated with respect to the sequence until there is no more unsearched search sequences. As the search result of the sequence 3-2, the feature vector {1, 0, 1} as shown in FIG. 10 is obtained.

Next, the feature vector creation unit 22 calculates a weight vector by summing the respective dimensional values of the feature vector with reference to the structures 3-1 and 3-2 obtained as described above (A35-1). Here, since the feature vector with reference to the structure 3-1 is {1, 1, 0} and the feature vector with reference to the structure 3-2 is {1, 0, 1}, the feature vector creation unit 22 calculates {2, 1, 1} as the weight vector. Thereafter, the feature vector creation unit 22 assigns weights to each of the previous feature vectors ({1, 1, 0} in structure 3-1 and {1, 0, 1} in structure 3-2) with the calculated weight vector ({2, 1, 2}) (A35-2). Here, when the assignment of weights is performed by multiplying each dimensional component of the previous feature vector by the corresponding dimensional component of the weight vector, {2, 1, 0} is obtained with respect to the structure 3-1 and {2, 0, 1} is obtained with respect to the structure 3-2. The feature vector creation unit 22 stores these values as the weighted feature vectors in place of the previous feature vectors (A35-3).

Thereafter, the feature vector creation unit 22 determines that there is no unsearched sequence with respect to all the sequences applied (sequences 3-1 to 3-2) (Step A36-2), and the results thereof are issued by the output device 4 (A37).

These results are the feature vectors obtained according to the present invention showing the features of the base sequences shown in FIG. 9.

In Example 1, the number of base sequences whose feature vector is created is one. Example 3 corresponds to the example in which plural base sequences are applied. In the present case, at the time of storing the search result as the vector by the feature vector creation unit 22, in consideration of the appearance frequency of the search sequence among the sequences, the value considered, instead of simple "1" or "0", can be stored as the feature vector. In Example 3, the value obtained by assigning weights to every dimensions of the feature vector is stored as the feature vector. However, as described above, the assignment of weights may be performed by other methods. Further, in accordance with weights, by eliminating the low-weighted-dimensions of the feature vector, the redundant dimensions of the feature vector may be compressed.

### Industrial Applicability

It is considered that the present invention is applicable to the statistical analysis in general including the problem of the classification of base sequences. With reference to the classification of the base sequences as an example, it is considered that, by efficiently classifying the base sequences, efforts in the step of obtaining aptamer can be reduced as well as the obtainment efficiency itself is increased by reducing subjective oversight of useful base sequences.

With that, the present invention is explained with reference to the preferred embodiment of the present invention. Although it is explained by showing the certain examples, it is obvious that any modifications and changes to the certain examples can be made without departing from the wide sprit and the scope of the present invention as recited in the claims. That is, it should not be interpreted that the present invention is limited to the explanation of the certain examples and the attached drawings.

## Claims

1. A feature extraction method comprising the steps of:
predicting a secondary structure of a base sequence applied; and
creating a feature vector based on a predicted secondary structure of the sequence.

2. The method according to claim 1, wherein the step of creating a feature vector comprises a step of extracting a loop site of the secondary structure.

3. The method according to claim 1 or 2, wherein the step of creating a feature vector comprises a step of forming a search sequence.

4. The method according to claim 3, wherein the search sequence is composed of residues selected from the group consisting of adenine, cytosine, guanine, and uracil or thymine or composed of the residues and a mismatch.

5. The method according to any one of claims 1 to 4, wherein the step of creating a feature vector comprises a step of searching the presence or absence of a loop site having a sequence of a search sequence.

6. The method according to any one of claims 1 to 5, wherein the step of creating a feature vector comprises a step of assigning weights to a search result with respect to a search sequence.

7. The method according to claim 6, where in the step of assigning weights, the number of loop sites having a sequence of a search sequence is calculated.

8. The method according to any one of claims 1 to 7, the step of creating a feature vector comprises a step of storing a search result with respect to a search sequence as the feature vector.

9. A feature extraction apparatus comprising:
a secondary structure prediction unit for predicting a secondary structure of a base sequence applied; and
a feature vector creation unit for creating a feature vector based on a predicted secondary structure of the sequence.

10. The apparatus according to claim 9, wherein the feature vector creation unit extracts a loop site of the secondary structure.

11. The apparatus according to claim 9 or 10, wherein the feature vector creation unit forms a search sequence.

12. The apparatus according to claim 11, wherein the search sequence is composed of residues selected from the group consisting of adenine, cytosine, guanine, and uracil or thymine or composed of the residues and a mismatch.

13. The apparatus according to any one of claims 9 to 12, wherein the feature vector creation unit searches the presence or absence of a loop site having a sequence of a search sequence.

14. The apparatus according to any one of claims 9 to 13, wherein the feature vector creation unit assigns weights to a search result with respect to a search sequence.

15. The apparatus according to claim 14, wherein the assignment of weights is performed by calculating the number of loop sites having a sequence of a search sequence.

16. The apparatus according to any one of claims 9 to 11, wherein the feature vector creation unit stores a search result with respect to a search sequence as the feature vector.

17. A feature extraction program performing the steps of:
predicting a secondary structure of a base sequence applied; and
creating a feature vector based on a predicted secondary structure of the sequence.

18. The feature extraction program according to claim 17, wherein the step of creating a feature vector comprises a step of extracting a loop site of the secondary structure.

19. The feature extraction program according to claim 17 or 18, wherein the step of creating a feature vector comprises a step of forming a search sequence.

20. The feature extraction program according to claim 19, wherein the search sequence is composed of residues selected from the group consisting of adenine, cytosine, guanine, and uracil or thymine or composed of the residues and a mismatch.

21. The feature extraction program according to any one of claims 17 to 20, wherein the step of creating a feature vector comprises a step of searching the presence or absence of a loop site having a sequence of a search sequence.

22. The feature extraction program according to any one of claims 17 to 21, wherein the step of creating a feature vector comprises a step of assigning weights to a search result with respect to a search sequence.

23. The feature extraction program according to claim 22, where in the step of assigning weights, the number of loop sites having a sequence of a search sequence is calculated.

24. The feature extraction program according to any one of claims 17 to 23, wherein the step of creating a feature vector comprises a step of storing a search result with respect to a search sequence as the feature vector.
